Europäisches Patentamt

European Patent Office

Office européen des brevets

(19)

(11) Publication number: **0 160 151**
**A2**

(12) **EUROPEAN PATENT APPLICATION**

(21) Application number: **84401780.6**

(22) Date of filing: **07.09.84**

(51) Int. Cl.⁴: **A 01 N 25/18**
**A 61 L 9/12**

(30) Priority: **11.04.84 JP 72399/84**

(43) Date of publication of application:
**06.11.85 Bulletin 85/45**

(84) Designated Contracting States:
**DE GB IT**

(71) Applicant: **Shin-Etsu Chemical Co., Ltd.**
**6-1, Ohtemachi 2-chome**
**Chiyoda-ku, Tokyo100(JP)**

(72) Inventor: **Nagura, Shigehiro**
**2-1-4-7, Minato-cho**
**Joetsu-shi Niigata-ken(JP)**

(72) Inventor: **Yamamoto, Akira**
**3-8-11, Higashishiro-cho**
**Joetsu-shi Niigata-ken(JP)**

(72) Inventor: **Ogawa, Kinya**
**1-7-32, Shiboku Miyamae-ku**
**Kawasaki-shi Kanagawa-ken(JP)**

(74) Representative: **Armengaud, Alain et al,**
**Cabinet ARMENGAUD AINE 3 Avenue Bugeaud**
**F-75116 Paris(FR)**

(54) A composite body for sustainedly releasing vapor of a vaporizable active substance and a method for the preparation thereof.

(57) The invention provides a vapor dispenser for the emission of a vaporizable substance exhibiting an activity in the atmosphere in a trace concentration, such as an insectan sex pheromone used in pest control, at a controlled uniform rate, a large number of which can be distributed in the field with minimum labor, for example, by tying to and hanging on the twigs of tea plants in a tea garden. The inventive vapor dispenser comprises a plastic-made tubular body having a specific cross sectional area of the pore and a specific outer circumferential length filled with the vaporizable substance and a coated or uncoated metal wire bonded to the tubular body side-by-side, said metal having a Brinell hardness of 65 or smaller, to facilitate tying works and stability of the dispenser tied and hung on the twigs by virtue of the moderate deformability and shape-retainability of the wire.

FIG. Ia

A COMPOSITE BODY FOR SUSTAINEDLY RELEASING VAPOR OF

A VAPORIZABLE ACTIVE SUBSTANCE AND A METHOD

FOR THE PREPARATION THEREOF

BACKGROUND OF THE INVENTION

The present invention relates to a vapor dispenser or a composite body for releasing the vapor of an active vaporizable substance sustainedly or at a controlled rate with stability over a long period of time and a method for the preparation thereof.

In accordance with the recent progress in the biologically active substances which may exhibit their activity in the form of vapor in a trace concentration in the atmosphere, such as perfumes, insect pheromones, certain kinds of medicines and agricultural chemicals, it is eagerly desired as an important practical problem to develop an efficient means for releasing or emitting the vapor of such a substance into the atmosphere sustainedly or at a controlled rate with stability over a long period of time.

In this regard, several methods using a specific vapor dispenser have been proposed hitherto. For example, U.S. Patents No. 3,539,465 and No. 3,577,515 teach a method of using microcapsules containing the vaporizable susbstance therein. This method is not quite satisfactory due to the relatively large loss of the vaporizable substance in the course of the preparation of the microcapsules containing the same and also due to the difficulty encountered when a uniform sustained releasability of the vaporizable substance is desired of the microcapsules over a long period of, for example, 1 month or longer. Alternatively, U.S. Patent No. 4,017,030 teaches a hollow fiber impregnated with the vaporizable active substance filling the pore of the hollow fiber. A problem in this method is the difficulty in the control of the vaporizing velocity of the vaporizable active substance through the walls of the hollow fiber. In addition, this method of using hollow fibers is not applicable when the volume of the vaporizable substance is relatively large due to the limited pore volume of the hollow fibers.

Apart from the above described problem of the sustained releasability of the vaporizable substance into the atmosphere from the vapor-releasing body containing the vaporizable substance, another difficult problem must be solved when the vapor-releasing body is to be used in an open field with an object, for example, to distribute an insectan sex pheromone over the field in the population control of pests in the agriculture or forestry. That is, a large number of such vapor-releasing bodies should be ditributed over the field easily with as little as possible labor consumption and the once distributed vapor-releasing bodies or vapor dispensers in the field should be left at the position with stability withstanding any strong windy or stormy conditions of the weather.

## SUMMARY OF THE INVENTION

It is therefore an object of the present invention to provide a novel and improved sustainedly vapor-releasing body of a vaporizable substance exhibiting activity in the vaporized form in the atmosphere which can be easily and economically prepared and is capable of releasing the vapor of the active substance contained therein at a controlled rate over a long period of time to be freed from the disadvantages and problems in the above mentioned microcapsule-type or hollow fiber-type vapor-releasing bodies in the prior art.

Another object of the invention is to provide a sustainedly vapor-releasing body which, in addition to the above mentioned characteristics for the release of the vapor of the active substance, can easily be distributed over the field, where the effect of the vaporizable active substance is desired, with great saving of the labor consumption and, once distributed in the field, can be left there with stability withstanding any adverse conditions of the weather.

A further object of the invention is to provide a method for the preparation of a sustainedly vapor-releasing body or vapor dispenser satisfying the above described objects.

Thus, the sustainedly vapor-releasing composite body or vapor dispenser of the invention for releasing the vapor of a vaporizable substance into the atmosphere comprises:

(a) a tubular body made of a plastic resin having an inner diameter in the range from 0.5 to 4 mm, an outer circumferential length in the range from 2 to 20 mm and a length in the range from 50 to 1000 mm;

(b) a wire having a diameter in the range from 0.5 to 1.5 mm and a length substantially equal to the length of the tubular body as the component (a) and made of a metal or an alloy having a Brinell hardness not exceeding 65, the wire being uncoated or coated with a plastic resin and adhesively bonded side-by-side to the tubular body as the component (a); and

(c) a vaporizable substance capable of exhibiting activity in the atmosphere when present in the form of a vapor and at least partially filling the pore of the tubular body as the component (a).

Accordingly, the method of the present invention for the preparation of a sustainedly vapor-releasing composite body or vapor dispenser as defined above comprises the steps of:

(i) adhesively bonding a wire of a continuous length having a diameter in the range from 0.5 to 1.5 mm and made of a metal or alloy having a Brinell hardness not exceeding 65, said wire being uncoated or coated with a plastic resin, side-by-side to a tubular body of a continuous length made
of a plastic resin and having an inner diameter in the range
from 0.5 to 4 mm and an outer circumferential length in the
range from 2 to 20 mm;

(ii) filling the pore of the tubular body with a vaporizable
substance; and

(iii) cutting the tubular body together with the wire adhe-
sively bonded thereto in unit lengths in the range from 50
to 1000 mm, the step (iii) optionally preceding the step
(ii).


## BRIEF DESCRIPTION OF THE DRAWING

FIGURES 1a and 1b are each a perspective view showing
the cross sectional end of the tubular body and the uncoated
or coated wire, respectively, adhesively bonded thereto
side-by-side.


FIGURES 2a to 2d each illustrate a different cross section of the tubular body of the vapor dispenser prepared and
tested in Example 2.


FIGURE 3 is a graph showing the amount of the released
vapor as a function of the days of exposure in Example 2.


## DETAILED DESCRIPTION OF THE PREFERRED EMBODIMENTS

As is understood from the above description, the main
part of the sustainedly vapor-releasing composite body or the vapor dispenser of the invention is the tubular body of a plastic resin having the specified dimensions. The plastic tubular body should have an inner diameter in the range from 0.5 tp 4 mm or, preferably, from 0.8 to 3 mm, an outer circumferential length in the range from 2 to 20 mm and a length in the range from 50 to 1000 mm. Assuming the cross sectional area of the pore of the tubular body being S $mm^2$ and the circumferential length being A mm, the ratio of A/S should preferably be in the range from 2 to 20 $(mm^{-1})$ or, more preferably, from 4 to 15 $(mm^{-1})$. These conditions relative to the cross section of the tubular body are important in order that the vapor dispenser prepared thereof may have a good balance between the controlled sustained vapor releasability and the mechanical strength or performance. When the cross section of the tubular body is not circular or is of an irregular form, the inner diameter here implied is the inner diameter of a circular tubular body having the same cross sectional area of the pore as that of the tubular body in question. Accordingly, it may better be called an "inner diameter equivalent" in such a case.

It is of course that a tubular body having an inner diameter smaller than 0.5 mm can contain no sufficiently large volume of the vaporizable substance therein per unit length and, in addition, the rate of vapor emission from such a fine tubular body may be excessively large at the initial stage of exposure with rapid decrease within a relatively short time so that the uniformity cannot be ensured in the rate of vapor release. On the other hand, a tubular body having an inner diameter exceeding 4 mm is undesirable due to the unduly low rate of vapor emission therefrom, in particular, at the late stage of the atmospheric exposure. The above mentioned A/S ratio is also of importance since the rate of vapor emission of the vaporizable substance would be too small when the A/S ratio is smaller than 2 $mm^{-1}$. In particular, the rate of vapor release from a tubular body having such a cross sectional dimension is too low to be practical when about a half amount of the vaporizable substance originaly contained therein has been lost by dissipation into the atmosphere. When the A/S ratio is larger than 20 $mm^{-1}$, on the other hand, the rate of vapor release through the walls of the tubular body would be too high with rapid decay of the rate in the lapse of time. When the rate of vapor release is plotted on a graph as a function of time, the curve for the rate of vapor release from such a tubular body follows about the same curve as from a vapor dispenser prepared by shaping a blend of the plastic resin with the vaporizable substance so that the desired object of the invention cannot be achieved.

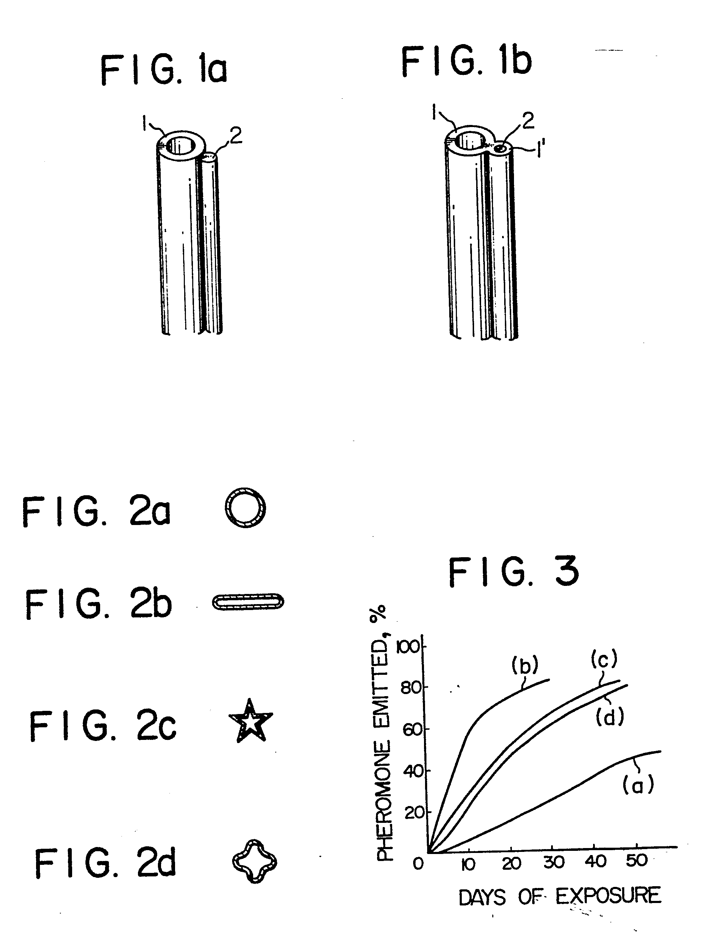

The cross sectional configuration of the tubular body is not limited to a circular one but may be square, astral and the like non-circular forms. Practically, the cross section is preferably circular, elliptic, wavy, astral or the like form. Wavy or astral cross sections are suitable for a tubular body having a relatively large cross sectional area while circular or elliptic cross sections are preferred in a tubular body having a relatively small cross sectional area. FIGURES 2a to 2d of the accompanying drawing show some of the possible cross sectional configurations including a circular (FIGURE 2a), flattened (FIGURE 2b), astral (FIGURE 2c) and quaternarily wavy (FIGURE 2d) forms though not limited thereto.

The plastic resin of which the tubular body is formed is not limited to a particular kind of polymers but may be exemplified by polyethylene, polypropylene, copolymers of ethylene and vinyl acetate, polyvinyl chloride resin, cellulose acetate, formalized polyvinyl alcohol and the like. When the vaporizable substance contained in the tubular body is a compound belonging to alcoholic and carboxylic acid compounds, the material of the tubular body is preferably a cellulose acetate or a formalized polyvinyl alcohol and, when the vaporizable substance is a compound belonging to aromatic compounds, ester compounds, aldehydes, ketones or hydrocarbons, the material of the tubular body is preferably a polyethylene or a copolymer of ethylene and vinyl acetate or, in particular, a high-density polyethylene from the standpoint of obtaining a high uniformity in the rate of vapor release. When the plastic resin is transparent, an

- 9 -

advantage is obtained that a visual means is provided for checking the rate of vapor release and the amount of the vaporizable substance remaining in the tubular body. Such an advantage is further increased when the vaporizable substance contained in the tube is colored, if possible. When the vaporizable substance contained in the tubular body is susceptible to and decomposed by the influence of light or, in particular, ultraviolet, on the other hand, it is preferable that the plastic resin is colored by incorporating a coloring agent such as a dye or pigment or an ultraviolet absorber is blended therein although stabilization of the vaporizable substance may be achieved by admixing the vaporizable substance with an antioxidant or a stabilizer.

In the inventive vapor dispenser or sustainedly vapor-releasing body, a wire of a metal or alloy is adhesively bonded side-by-side to the above mentioned tubular body of a plastic resin. The object of using such a metal wire is to impart the vapor dispenser with a shape-retainability when it is deformed since a tubular body of a plastic resin having more or less of elastic resilience can hardly retain its deformed configuration to cause difficulties when it is desired to manually hang the vapor dispenser on a twig of a tree and to keep the dispenser thus once hung on a tree with stability or without falling off. In this regard, the metal or alloy for the wire should have a Brinell hardness not excessively high. This condition is particularly important when a large number of such vapor dispensers are distributed over a tea garden, orchard and the like agricultural field since use of a scissors or shears in such a field for tea-picking or pruning of fruit trees unavoidably has a danger of inadvertently cutting off the vapor dispenser to cause nicking of the edges of the scissors or shears unless the Brinell hardness of the wire is sufficiently low. In these regards, the Brinell hardness of the metal or alloy for the wire should be 65 or lower so that the danger of eventual nicking on the edged tools is avoided and the works for hanging the vapor dispenser can be performed very efficiently without fatigue on the workers' hands in addition to the reliability or stability of the vapor dispenser, for example, hanging on the tree in the field without falling down by any strong wind. Preferable materials for the wire include aluminum, copper and alloys thereof while steels and the like material having a larger Brinell hardness are undesirable.

The wire should preferably have a diameter in the range from 0.5 to 1.5 mm because a wire having a diameter smaller than 0.5 mm cannot impart a sufficient shape-retainability to the deformed plastic-made tubular body by outbalancing the elastic resilience of the tubular body while a wire having a diameter larger than 1.5 mm may cause some inconvenience due to the excessively large resistance against plastic deformation of the wire and hence fatigue in the workers' hands in addition to the economic disadvantage as a natural consequence of the use of an unnecessarily large wire. If desired, the wire may be coated in a sheath-and-core manner with a plastic resin or the like coating material in a suitable thickness when increased corrosion resistance and easiness of adhesive bonding to the tubular body are desired of the wire. The coating material is preferably the same plastic resin as that of the tubular body containing the vaporizable substance so that the composite body can be prepared by the techniques of co-extrusion with the wire. FIGURE 1b of the accompanying drawing is a perspective view of such a composite body showing an end surface in which the metal wire 2 is provided with a coating layer 1' and adhesively bonded to the tubular body 1 through the coating layer 1' while FIGURE 1a is a similar perspective view in which the wire 2 is not provided with a coating layer and directly bonded to the tubular body 1.

The uncoated wire and the tubular body should be adhesively bonded together side-by-side by melting the plastic resin of the tube or by use of an adhesive. This does not mean that the wire should be substantially in parallel to the axis of the tubular body but the only requirement is that the running direction of the wire is virtually in the length-wise direction of the tubular body. For example, the wire and the tubular body may be twisted together each around the other. Further, it is not always necessary that the wire is adhe-sively bonded to the tubular body over the whole length thereof but they are bonded together spot-wise at several points with suitable intervals. Further, the wire and the tubular body may have different lengths from each other. It is optional that two or more wires are bonded to a tubular body each side-by-side or two or more tubular bodies are adhesively bonded to one and the same wire.

Following is a description of a typical method for the preparation of the above described vapor dispenser or sustainedly vapor-releasing composite body of the invention. Thus, a continuous-length tube of a plastic resin of a desired type and having a desired cross section is adhesively bonded together to a continuous-length metallic wire, which may be coated or uncoated, by a suitable method. It is a convenient way to undertake a technique of co-extrusion in which a composite of a tubular body and a plastic-coated wire is extruded in one shot to give a side-by-side composite body of a plastic tube and a plastic-coated wire having a cross section illustrated in FIGURE 1b.

The thus prepared side-by-side composite body is cut in a suitable length and the vaporizable substance in a liquid form is pressurized into the tubular body at one open end by evacuating the air inside the tubular body from the other open end until the pore of the tubular body is completely filled with the liquid. Thereafter, the composite body filled with the liquid of the vaporizable substance is welded at spots with suitable intervals by use of, for example, a high frequency welding machine and the like followed by cutting at the welded portions into a number of the vapor dispensers each having a unit length of 50 to 1000 mm and sealed at both ends. It is of course optional that the impregnation of the tubular body with the vaporizable substance is preceded by cutting the continuous-length composite body of the tubular body and the wire into unit lengths.

In th following, examples are given to illustrate the invention in further detail.

Example 1.

A high-density polyethylene tube of 200 mm length having a circular cross section with an inner diameter of 0.9 mm and an outer diameter of 1.5 mm corresponding to the A/S value of about $7.4$ $mm^{-1}$ was filled with 95 mg of cis-11-tetradecenyl acetate known as an insectan sex pheromone and the tube was sealed by welding at both ends. An aluminum wire of 0.9 mm diameter having a Brinell hardness of 46 and coated with a high-density polyethylene was bonded by melting to the pheromone-impregnated polyethylene tube side-by-side to give a composite body such as that illustrated in FIGURE 1b to serve as a vapor dispenser of the pheromone compound.

The thus prepared vapor dispenser was hung on a twig of a tea tree in a tea garden by tying utilizing the deformability and shape-retainability of the aluminum wire and exposed to the open air for 60 days at temperatures of 22 to 28 °C with periodical weighing to determine the lost amount of the pheromone compound by emission to find that the rate of vapor releasing from the vapor dispenser was quite uniform over the period. Meanwhile, despite the strong wind of 10 meters/second or faster during the test period, the vapor dispenser never fell down or was blown away from the twig.

For comparison, a similar vapor dispenser to the above was prepared by replacing the aluminum wire with a steel wire of the same diameter having a Brinell hardness of 178. This comparative vapor dispenser could be tied to the twig of the tea tree with considerable difficulties due to the resilience of the steel wire. When this steel wire-bearing vapor dispenser was cut with tea-picking scissors, the edges of the scissors were sometimes nicked while the aluminum wire-bearing vapor dispenser could be cut off with the scissors without nicking on the scissor blades at all.

For further comparison, the same polyethylene tube without a metal wire was filled with the insect pheromone but the thus prepared wire-free vapor dispenser could be hung on the twig of a tea tree with extreme difficulties due to the elastic rsilience of the polyethylene tube and the once tied polyethylene tube was readily untied to fall down.

Example 2.

A copolymer of ethylene and vinyl acetate was shaped into tubes (a) to (d) having different cross sections as illustrated in FIGURES 2a to 2d, respectively, and each having a length of 200 mm. The cross sectional area of the pore S in $mm^2$ and the circumferential length A in mm of each tube as well as the A/S value thereof in $mm^{-1}$ were as tabulated below.

| Tube | S, $mm^2$ | A, mm | A/S, $mm^{-1}$ |
|------|-----------|-------|----------------|
| (a)  | 7.0       | 21.7  | 3.1            |
| (b)  | 7.2       | 150   | 20.9           |
| (c)  | 6.7       | 44.9  | 6.7            |
| (d)  | 7.1       | 43.3  | 6.1            |

A copper wire of 0.8 mm diameter having a Brinell hardness of 48 and coated with a copolymer of ethylene and vinyl acetate was bonded by melting of the coating layer to each of the tubes (a) to (d) as illustrated in FIGURE 1b and each of the thus prepared composite bodies was filled with 1.1 g of cis-11-tridecenyl acetate known as an insectan sex pheromone followed by sealing at both ends to give a vapor dispenser.

These vapor dispensers were exposed to atmospheric air

at 20 ºC for 50 days with periodical weighing to determine the lost amount of the pheromone compound. The results were as shown in the graph of FIGURE 3 and quite satisfactory in the tubes (c) and (d).

Example 3.

A continuous length composite body was prepared by bonding together a tube of a high-density polyethylene having an inner diameter of 0.65 mm and an outer diameter of 0.98 mm corresponding to the A/S value of about 9.3 $mm^{-1}$ and a wire of 0.6 mm diameter made of an aluminum alloy AA5154 having a Brinell hardness of 58 and coated with the same polyethylene by the techniques of co-extrusion followed by cutting into a 500 meters length.

The thus prepared composite tube was filled with 7,11-hexadecadienyl acetate known as an insectan sex pheromone introduced from one end under a pressure of 3.0 $kg/cm^2G$ with simultaneous evacuation of the tube at the other end and welded by high frequency heating at spots with 10 cm intervals followed by cutting at the welded portions into 10-cm long pieces each sealed at both ends to serve as a vapor dispenser of the pheromone compound. No nicking of the edges was noted on the cutting tool used in the above mentioned cutting works. The works of hanging and unhanging of these vapor dispensers on and from cotton plants could be performed without difficulties by virtue of the moderate deformability and shape-retainability of the wire and the dispensers once hung on the cotton plant were never lost even under a stormy weather for a period of 70 days at 28 to 33 °C continuing stable emission of the pheromone vapor.

For comparison, similar vapor dispensers were prepared by replacing the above used wire of aluminum alloy with a wire of mild steel of the same diameter having a Brinell hardness of 83. Cutting of the composite tube bearing the mild steel wire was performed with several times of nicking of the edges on the cutting tool. Mounting of these comparative vapor dispensers on cotton plants could be performed only with great difficulties almost to inhibit manual working.

WHAT IS CLAIMED IS:

1.   A sustainedly vapor-releasing composite body for controlled emission of a vaporizable substance which comprises:

(a) a tubular body made of a plastic resin having an inner diameter in the range from 0.5 to 4 mm, an outer circumferential length in the range from 2 to 20 mm and a length in the range from 50 to 1000 mm;

(b) a wire having a diameter in the range from 0.5 to 1.5 mm and a length substantially equal to the length of the tubular body as the component (a) and made of a metal or an alloy having a Brinell hardness not exceeding 65, the wire being uncoated or coated with a plastic resin and adhesively bonded side-by-side to the tubular body as the component (a); and

(c) a vaporizable substance capable of exhibiting activity in the atmosphere when present in the form of a vapor and at least partially filling the pore of the tubular body as the component (a).

2.   The sustainedly vapor-releasing composite body as claimed in claim 1 wherein the ratio of the outer circumferential length of the tubular body in mm to the cross sectional area of the pore of the tubular body in $mm^2$ is in the range from 2 to 20 $mm^{-1}$.

3.   The sustainedly vapor-releasing composite body as claimed in claim 1 wherein the plastic resin of the tubular body is a high-density polyethylene.

4. The sustainedly vapor-releasing composite body as claimed in claim 1 wherein the metal or alloy of the wire is selected from the group consisting of aluminum, copper and alloys mainly composed of aluminum or copper.

5. A method for the preparation of a sustainedly vapor-releasing composite body for controlled emission of a vaporizable substance which comprises the steps of:

(i) adhesively bonding a wire of a continuous length having a diameter in the range from 0.5 to 1.5 mm and made of a metal or alloy having a Brinell hardness not exceeding 65, said wire being uncoated or coated with a plastic resin, side-by-side to a tubular body of a continuous length made of a plastic resin and having an inner diameter in the range from 0.5 to 4 mm and an outer circumferential length in the range from 2 to 20 mm;

(ii) filling the pore of the tubular body with a vaporizable substance;

(iii) sealing the tubular body by welding at spots with intervals in the range from 50 to 1000 mm; and

(iv) cutting the tubular body together with the wire adhesively bonded thereto at the sealed portions into unit lengths.

# FIG. 1a

# FIG. 1b

# FIG. 2a

# FIG. 2b

# FIG. 2c

# FIG. 2d

# FIG. 3

PHEROMONE EMITTED, %

DAYS OF EXPOSURE